# EUROPEAN PATENT SPECIFICATION

(11) **EP 4 089 168 B1**
(45) Date of publication and mention of the grant of the patent: **15.05.2024**
(21) Application number: 20912092.2
(22) Date of filing: 10.12.2020
(51) Int. Cl.: C12N 15/11, C12N 15/87, C07K 5/083, A61K 48/00

(54) **METHOD FOR PREPARING MRNA-GALNAC TARGETING MOLECULE, IN VIVO DELIVERY SYSTEM THEREFOR, AND USE THEREOF**
VERFAHREN ZUR HERSTELLUNG EINES MRNA-GALNAC-ZIELMOLEKÜLS, IN-VIVO-ABGABESYSTEM DAFÜR UND DESSEN VERWENDUNG
PROCÉDÉ DE PRÉPARATION D'UNE MOLÉCULE DE CIBLAGE ARNM-GALNAC, SYSTÈME D'ADMINISTRATION IN VIVO ASSOCIÉ ET SON UTILISATION

(30) Priority: 10.01.2020 CN 202010027183
(43) Date of publication of application: 16.11.2022
(73) Proprietor: Shenzhen Rhegen Biotechnology Co., Ltd., Shenzhen, Guangdong 518057 (CN)
(72) Inventor: HU, Yong, Shenzhen, Guangdong 518057 (CN); ZHANG, Miaomiao, Shenzhen, Guangdong 518057 (CN)
(74) Representative: Mewburn Ellis LLP
(86) International application number: PCT/CN2020/135203
(87) International publication number: WO 2021/139474

(56) References cited:
- WO-A1-2017/212009
- WO-A1-2019/140102
- CN-A- 103 282 502
- CN-A- 105 658 797
- CN-A- 105 899 666
- CN-A- 108 271 387
- CN-A- 108 949 772
- YUANYU HUANG: "Preclinical and Clinical Advances of GalNAc-Decorated Nucleic Acid Therapeutics", MOLECULAR THERAPY-NUCLEIC ACIDS, vol. 6, 1 March 2017 (2017-03-01), pages 116-132, XP055485332, US ISSN: 2162-2531, DOI: 10.1016/j.omtn.2016.12.003
- Shintaro Fumoto, Koyo Nishida: "Methods for Evaluating the Stimuli-Responsive Delivery of Nucleic Acid and Gene Medicines", Chemical and Pharmaceutical Bulletin, vol. 65, no. 7, 1 July 2017 (2017-07-01), pages 642-648, XP055827466, ISSN: 0009-2363, DOI: 10.1248/cpb.c17-00096

## Description

The present application claims priority of Chinese Patent Application No. CN202010027183.1, entitled "METHOD FOR PREPARING MRNA-GALNAC TARGETING MOLECULE, IN VIVO DELIVERY SYSTEM THEREFOR, AND USE THEREOF" and filed with China National Intellectual Property Administration on January 10, 2020, the entire content of which is incorporated by reference herein.

### TECHNICAL FIELD

The present invention belongs to the technical field of molecular biology, and in particular relates to a method for preparing an mRNA-GalNAc targeting molecule, an *in vivo* delivery system therefor and use thereof.

### BACKGROUND OF THE INVENTION

At present, delivering mRNAs into cells can be achieved by different methods, such as electroporation, sonoporation, microinjection, or compound transfection. However, these methods cannot meet clinical needs in term of cytotoxicity and biological safety, and have certain difficulties in clinical transformations.

An asialoglycoprotein receptor (ASGPR) is an abundant hetero-oligomer endocytic receptor, mainly exists on a surface of cell membrane of liver parenchymal cells facing the sinusoid, and has specificity to sugar. Since exposed secondary ends are galactose residues after terminal sialic acids of various glycoproteins are removed via enzyme hydrolysis or acidolysis, the sugar-binding specificity of ASGPR actually acts on galactosyl groups and ASGPR is also called as a galactose specific receptor. Glycoproteins with non-reductive galactose (Gal) or N-acetylgalactosamine (GalNAc) residues at the ends can be recognized by ASGPR, and the affinity of ASGPR binding to GalNAc is dozens of times (about 50 times) higher than that to Gal. It has been observed in studies that the tri-tentacle clustered GalNAc conjugates can increase the affinity of GalNAc with hepatic parenchymal cells by about 50 times.

After years of continuous research and development, the GalNAc-conjugated small interfering RNA (siRNA) has achieved high-efficiency targeted drug delivery in liver. Although the receptor has been discovered for many years, messenger RNA (mRNA) delivery systems based on the receptor and its ligands have failed to achieve a breakthrough because of problems such as failure to achieve high-efficiency coupling between GalNAc and mRNA by existing technical means, charge property of the mRNA-GalNAc conjugate during *in vivo* delivery, and efficiency of escape from endosome after endocytosed by a cell.

### SUMMARY OF THE INVENTION

In view of the above technical problems, the present invention discloses a method for preparing an mRNA-GalNAc targeting molecule, *in vivo* delivery system therefor and use thereof, realizing direct and high-efficiency coupling between mRNA and N-acetylgalactosamine, modifying charge property of the mRNA-GalNAc conjugate during *in vivo* delivery by a positively charged protein, and increasing its efficiency of escape from endosome.

For this, the technical solutions adopted by the present invention are as follows.

The present invention provides an mRNA-GalNAc targeting molecule, comprising an mRNA molecule that is linked to PolyA modified with an N-acetylgalactosamine at 3'-end, wherein a sequence of the mRNA molecule comprises a 5' cap and a target gene sequence.

Preferably, the sequence of the mRNA molecule further comprises 5'UTR and 3'UTR, wherein the 5'UTR comprises a Kozak sequence.

In the present invention, the sequence of the mRNA is formed by sequentially connecting the 5'cap, the 5'UTR sequence comprising the Kozak sequence, the target gene sequence, the 3'UTR sequence and the PolyA sequence.

Preferably, the mRNA molecule is consisted of uridine, cytosine, adenosine, guanosine and a chemically modified nucleoside, wherein the chemically modified nucleoside is selected from one or more of 2-fluoro-2-deoxyadenosine, 2-fluoro-2-deoxyuridine, 2-fluoro-2-deoxycytidine, 2-fluoro-2-deoxyguanosine, 2-fluoro-2-deoxy-5-methylcytidine, 2-fluoro-2-deoxy-pseudouridine, 2-fluoro-2-deoxy-N1-methyl-pseudouridine, 2-fluoro-2-deoxy-N7-methyl-guanosine, 2-fluoro-2-deoxy-5-methoxyuridine, 2-fluoro-2-deoxy-N4-acetylcytidine, 2-fluoro-2-deoxy-N6-methyladenosine, 5-methylcytidine, pseudouridine, N1-methyl-pseudouridine, N7-methyl-guanosine, 5-methoxyuridine, N4-acetylcytidine and N6-methyladenosine.

Preferably, the 5'cap structure is selected from one or more of Cap0, Cap1, Cap2, ARCA, inosine, N1-methyl-guanosine, 2'fluoro-guanosine, 7-deaza-guanosine, 8-oxo-guanosine, 2-amino-guanosine, LNA-guanosine, 2-azido-guanosine, 7-methyl-guanosine-5'-triphosphate-5'-adenosine, guanosine-5'-triphosphate-5'-adenosine, 7-methyl-guanosine-5'-triphosphate-5'-guanosine, guanosine-5'-triphosphate-5'-guanosine, and 7-methyl-guanosine-5'-triphosphate-5'-2-methoxyadenine-guanosine.

Preferably, the mRNA molecule is linked to the PolyA modified with an N-acetylgalactosamine at 3'-end through a splint DNA.

Preferably, the mRNA molecule is obtained by *in vitro* transcribing a plasmid comprising a DNA fragment, wherein the DNA fragment comprises a promoter, a target gene, and a first splint DNA sequence that are sequentially connected; and the PolyA fragment modified with an N-acetylgalactosamine at 3'-end has a 5'-end which is an RNA sequence that can be complementarily paired with a second splint DNA sequence, wherein the first splint DNA sequence and the second splint DNA sequence form a splint DNA.

Preferably, the first splint DNA sequence is as shown in SEQ ID No. 1, the second splint DNA sequence is as shown in SEQ ID No. 2, and the promoter is a T3 or T7 or SP6 promoter.

The present invention further provides a method for preparing an mRNA-GalNAc targeting molecule as described above, comprising the following steps:
Step S1, designing and synthesizing a plasmid vector having a promoter sequence and a target gene sequence;
Step S2, *in vitro* transcribing the plasmid vector of Step S1 as a template to obtain an mRNA molecule, wherein a sequence of the mRNA molecule comprises a 5'cap and the target gene sequence; and
Step S3, binding the mRNA molecule to a PolyA sequence modified with an N-acetylgalactosamine at 3'-end under the action of a ligase, to obtain the mRNA-GalNAc targeting molecule.

Preferably, in Step S1, the DNA sequence in the plasmid vector comprises the promoter sequence, the target gene sequence, and a sequence complementary to a first splint DNA sequence, that are sequentially connected.

The sequence of the mRNA molecule obtained in Step S2 comprises the 5'cap, the target gene sequence, and an RNA sequence corresponding to the sequence complementary to the first splint DNA sequence, that are sequentially connected.

In Step S3, the PolyA fragment modified with an N-acetylgalactosamine at 3'-end has a 5'-end which is an RNA sequence that can be complementarily paired with a second splint DNA sequence, wherein the first splint DNA sequence and the second splint DNA sequence form a splint DNA. In annealing reaction, the mRNA molecule with the sequence complementary to the first splint DNA sequence and the PolyA molecule with the sequence complementarily paired with the second splint DNA sequence complementarily bind to the splint DNA, respectively. A 3'-end hydroxyl group of the mRNA molecule is linked to a 5'-end phosphate group of the PolyA modified with an N-acetylgalactosamine under the action of a T4 DNA ligase. After treatment by a DNase, the mRNA targeting molecule modified with an N-acetylgalactosamine is obtained.

Further, in Step S3, the PolyA fragment modified with an N-acetylgalactosamine at 3'-end has a 5'-end which is an RNA sequence that can be complementarily paired with a second splint DNA sequence, wherein the first splint DNA sequence and the second splint DNA sequence form the splint DNA; and
in the absence of the splint DNA, directly coupling the mRNA molecule to the PolyA modified with an N-acetylgalactosamine at 3'-end has problems of a high mismatch rate and low linking efficiency. The technical solution of the present invention adopts a splint DNA, which can greatly improve efficiency and accuracy of linking. In annealing reaction, the mRNA molecule with the sequence complementary to the first splint DNA sequence and the PolyA molecule with the RNA sequence complementarily paired with the second splint DNA sequence complementarily bind to the splint DNA, respectively. A 3'-end hydroxyl group of the mRNA molecule is linked to a 5'-end phosphate group of the PolyA modified with an N-acetylgalactosamine under the action of a T4 DNA ligase. After treatment by a DNase, the mRNA targeting molecule modified with an N-acetylgalactosamine is obtained.

Preferably, the promoter is a T3 or T7 or SP6 promoter.

Preferably, the first splint DNA sequence includes, but is not limited to, the sequence as shown in SEQ ID No. 1, specifically 5'-taccggttag-3', and the second splint DNA sequence includes, but is not limited to, the sequence as shown in SEQ ID No. 2, specifically 5'-taatgagttt-3'.

The present invention further provides a pharmaceutical composition comprising the mRNA-GalNAc targeting molecule as described above and a pharmaceutically acceptable excipient.

The present invention further provides a use of the mRNA-GalNAc targeting molecule or the pharmaceutical composition as described above in preparation of a medicament for expressing a target polypeptide in a mammalian subject.

The present invention further provides an *in vivo* delivery system comprising the mRNA-GalNAc targeting molecule as described above and a positively charged protein molecule.

It has been found in researches that the directly coupled mRNA-GalNAc without charge neutralization cannot achieve tissue-targeting delivery *in vivo,* but can achieve only transfection to liver cells in an *in vitro* cell experiment. The delivery system of the present invention achieves *in vivo* delivery that does not rely on traditional liposomes and lipid nanoparticles, by using positively charged proteins, thereby improving the stability of mRNA-GalNAc *in vivo,* increasing the efficiency of escape from endosome after endocytosis, and achieving tissue and organ targeting delivery.

Preferably, the positively charged protein is at least one of protamine and human serum albumin. The technical solution of the present invention achieve tissue and organ targeting delivery *in vivo* by improving the stability of the mRNA-GalNAc *in vivo* by screening effective positively charged protein molecules and neutralizing the negative charge of the mRNA molecule itself. At present, applying positively charged proteins such as protamine and human serum albumin to nucleic acid delivery is mainly relied on coupling to vectors such as liposomes and lipid nanoparticles to realize their functions. The technical solutions of the present invention achieve tissue and organ targeting delivery *in vivo* that does not rely on traditional vectors such as liposomes and lipid nanoparticles by combining mRNA-GalNAc, protamine, and human serum albumin.

Preferably, the *in vivo* delivery system is consisted of protamine, human serum albumin and an mRNA targeting molecule modified with an N-acetylgalactosamine, and the molar ratio of protamine to human serum albumin is 1: (2.75-5.5) or 1: (6-20). Further preferably, the molar ratio of protamine to human serum albumin is 1:2.75-5.5. This technical solution can improve the stability and transfection efficiency of mRNA *in vivo.*

The present invention further provides a use of the mRNA-GalNAc targeting molecule as described above in preparation of an mRNA drug for specific drug delivery.

The present invention further provides a use of an mRNA-GalNAc targeting molecule as described above in targeted drug delivery *in vivo.* The 3'-end of the mRNA-GalNAc targeting molecule is linked to an N-acetylgalactosamine, and the mRNA-GalNAc targeting molecule specifically binds to sialoglycoprotein receptors on a surface of the liver cell through the N-acetylgalactosamine, which induces endocytosis and therefore allows the mRNA to enter into the cell for expression.

The mRNA-GalNAc targeting molecule of the present invention can be used in a drug delivery system. The 3'-end of the mRNA targeting molecule is linked to an N-acetylgalactosamine, and the mRNA-GalNAc targeting molecule specifically binds to sialoglycoprotein receptors on a surface of the liver cell through the N-acetylgalactosamine, which induces endocytosis and therefore allows the mRNA to enter into the cell for expression. In this way, the technical problem of targeting delivery of nucleic acid drugs during drug delivery is solved.

Compared with the prior art, the present invention has the following beneficial effects.

The technical solutions of the present invention achieve *in vivo* targeting delivery under the action of the positively charged proteins by directly linking the mRNA molecule expressing the target gene to the PolyA sequence coupled with GalNAc to synthesize the mRNA molecule with GalNAc at 3'-end, thereby realizing the purpose of targeted drug delivery in liver. The method of the present invention does not rely on traditional vectors such as liposomes and lipid nanoparticles, and is simpler and more reliable, solving the existing problem of coupling an mRNA to N-acetylgalactosamine and *in vivo* targeting delivery. Further, the efficiency and accuracy of linking can be greatly improved by the splint DNA. The positively charged protein molecule is used to neutralize the negative charge of the mRNA molecule itself to improve the stability of the mRNA-GalNAc *in vivo* and the efficiency of escape from endosome, thereby achieving improved tissue and organ targeting delivery of drugs *in vivo.*

### BRIEF DESCRIPTION OF THE DRAWINGS

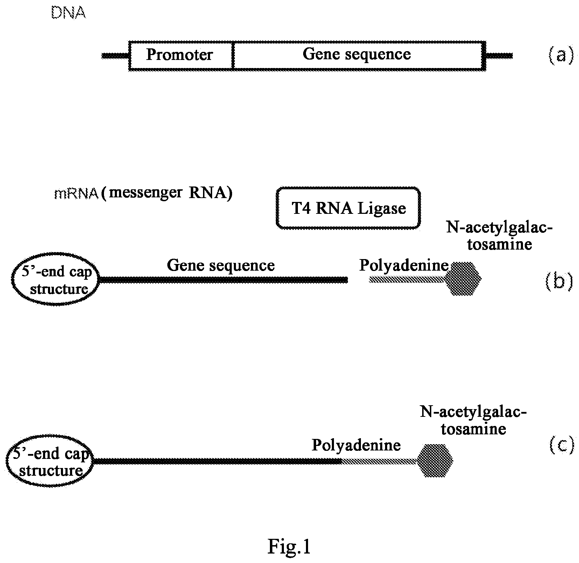

Figure 1 is a schematic flow chart showing the method for preparing an mRNA-GalNAc targeting molecule according to Example 1 of the present invention, wherein (a) is a structural schematic diagram showing the DNA fragment in the plasmid DNA and the mRNA molecule obtained by *in vitro* transcription; (b) is a schematic diagram showing the mRNA molecule and the PolyA modified with an N-acetylgalactosamine at 3'-end in a to-be-linked configuration; and (c) is a schematic diagram of the obtained mRNA-GalNAc targeting molecule.

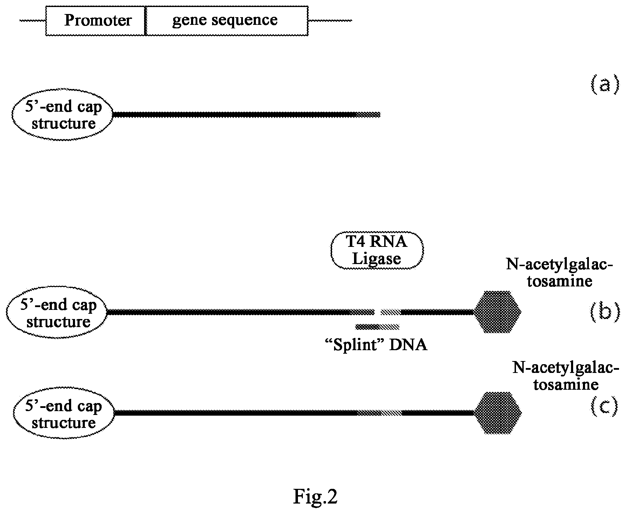

Figure 2 is a schematic flow chart showing the method for preparing an mRNA-GalNAc targeting molecule according to Example 2 of the present invention, wherein (a) is a structural schematic diagram showing the DNA fragment in the plasmid DNA and the mRNA molecule obtained by *in vitro* transcription; (b) is a schematic diagram showing the mRNA molecule linked to the PolyA modified with an N-acetylgalactosamine at 3'-end through the splint DNA; and (c) is a schematic diagram of the obtained mRNA-GalNAc targeting molecule.

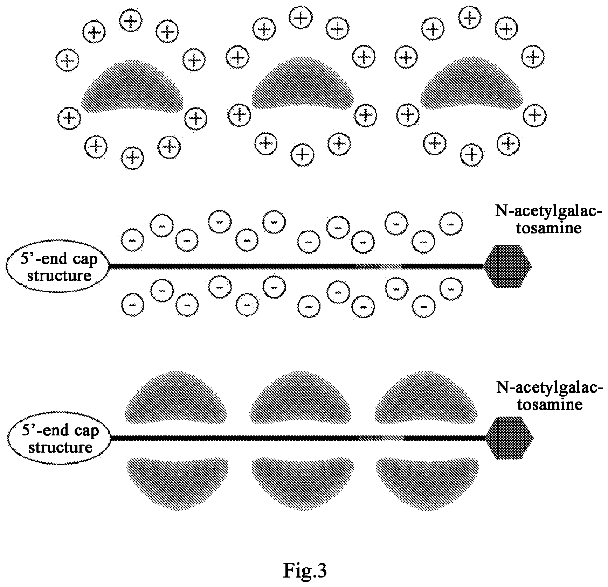

Figure 3 is a principle diagram showing preparation of the *in vivo* delivery system by interaction of the mRNA-GalNAc targeting molecule and the positively charged protein according to Example 2 of the present invention.

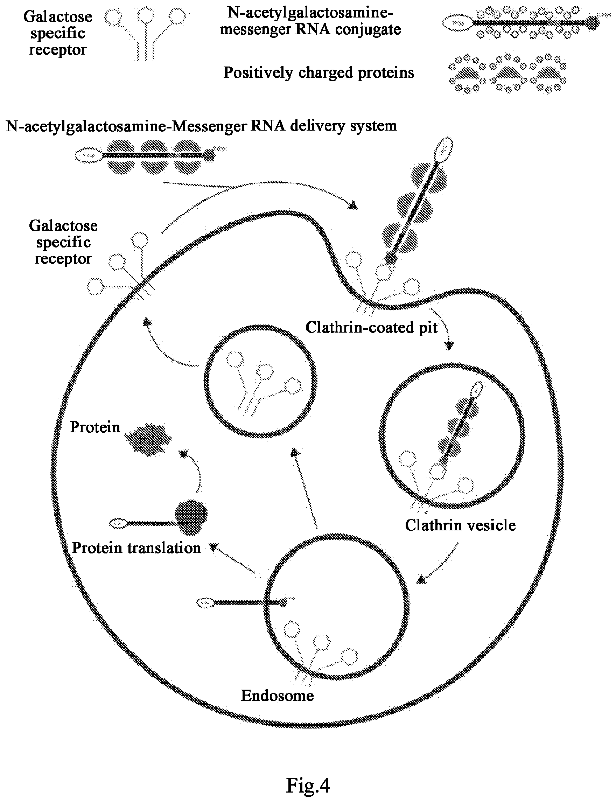

Figure 4 is a principle schematic diagram showing the *in vivo* delivery of the mRNA-GalNAc targeting molecule according to Example 2 of the present invention, wherein by binding to ASGPR on a surface of liver cells and inducing endocytosis, the GalNAc-mRNA conjugate allows the mRNA to enter into the cells and express the target gene protein after escaping from endosome and ribosomal translation.

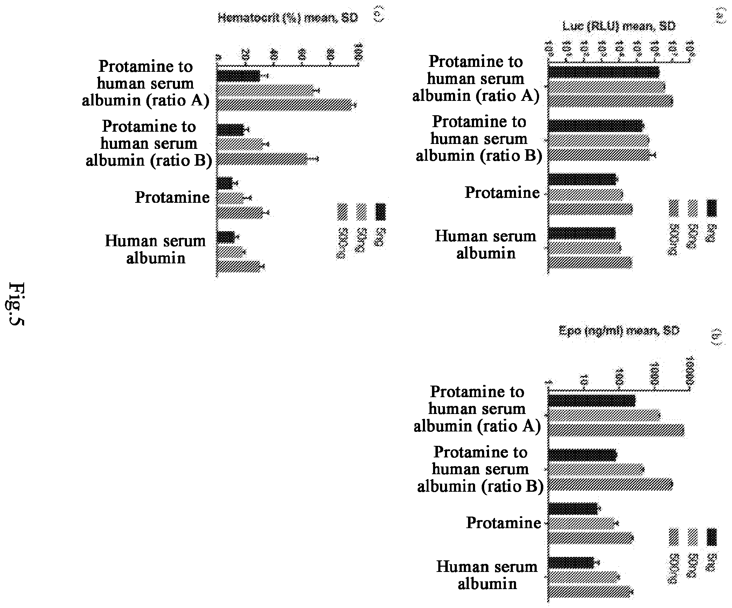

Figure 5 is a diagram showing comparison results in term of delivering the target gene to a liver tissue *in vivo* by the GalNAc-mRNA delivery systems according to Example 3 of the present invention, wherein (a) is the activity comparison result of using the delivery systems with different ratios of protamine to human serum albumin for delivering luciferase (Luc) *in vivo*; (b) is the comparison result of the delivery systems with different ratios of protamine to human serum albumin in term of erythropoietin (Epo); and (c) is the comparison result of animals' hematocrit obtained after using the delivery systems with different ratios of protamine to human serum albumin for delivering erythropoietin (Epo).

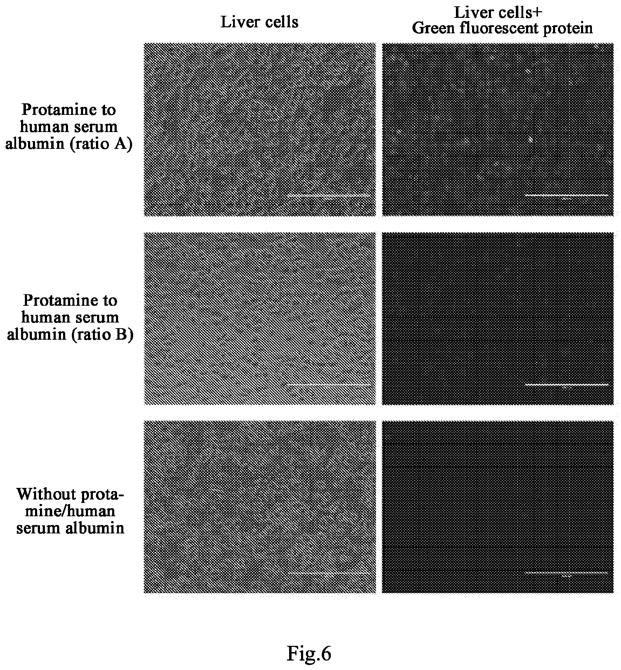

Figure 6 is a comparison schematic diagram showing expressions of green fluorescent protein (GFP) in liver cells obtained by the GalNAc-mRNA liver delivery systems with two different ratios of protamine to human serum albumin and a control delivery system without the positively charged proteins, according to Example 3 of the present invention.

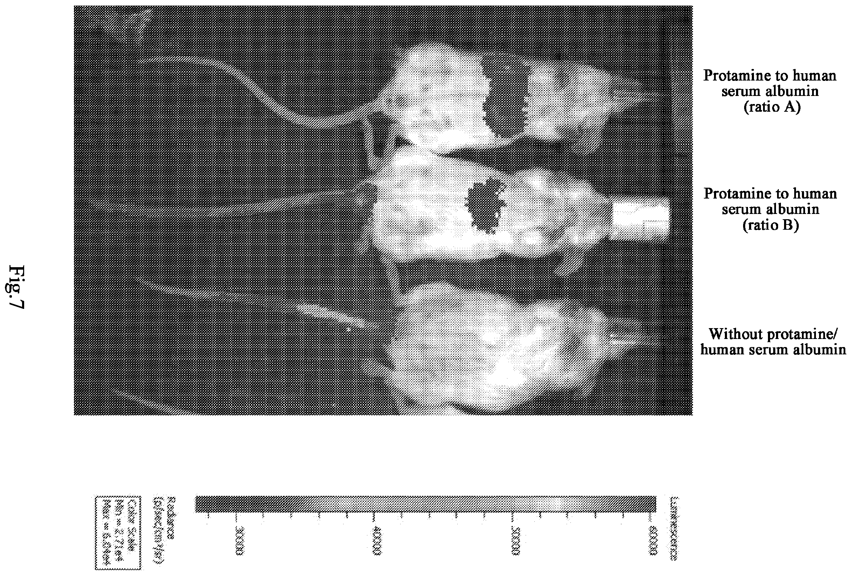

Figure 7 is a schematic diagram showing results of delivering luciferase (Luc) to the liver tissue *in vivo* by the GalNAc-mRNA liver delivery systems with different ratios of protamine to human serum albumin and the control delivery system, according to Example 4 of the present invention.

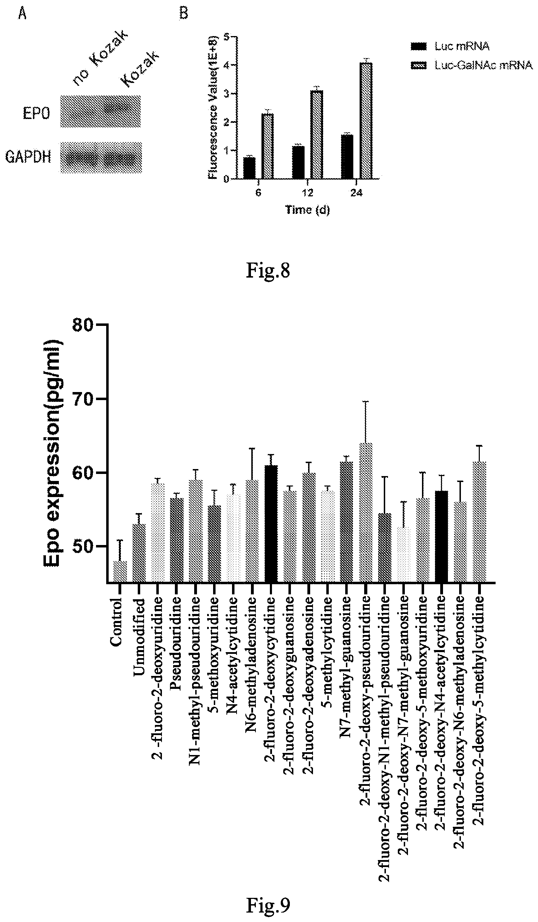

Figure 8 shows effect of the Kozak sequence and the GalNAc modification on expression efficiency of the mRNA according to Example 5 of the present invention, wherein A shows the expression efficiency after the cells are transfected with the EPO mRNAs with and without the Kozak sequence; and B shows the expression efficiency after intramuscularly injecting Luciferase mRNAs with and without GalNAc modification into mice.
Figure 9 is a concentration comparison of EPO proteins in blood obtained after intravenously injecting the EPO mRNAs containing different chemically modified bases into mice, according to Example 6 of the present invention.

### DETAILED DESCRIPTION OF THE INVENTION

The preferred Examples of the present invention will be described in further detail below.

### Example 1

Provided herein is an mRNA-GalNAc targeting molecule, which comprises an mRNA molecule that is linked to PolyA modified with an N-acetylgalactosamine at 3'-end, wherein a sequence of the mRNA molecule comprises a 5' cap and a target gene sequence.

As shown in Figure 1, the mRNA-GalNAc targeting molecule is prepared by the following steps.
Step S1, designing and synthesizing a plasmid vector having a promoter sequence and a target gene sequence;
Step S2, *in vitro* transcribing the plasmid vector of Step S1 as a template to obtain an mRNA molecule, wherein the sequence of the mRNA molecule comprises a 5'cap and a target gene sequence; and
Step S3, binding the mRNA molecule to the PolyA sequence modified with an N-acetylgalactosamine at 3'-end under the action of a ligase, to obtain the mRNA-GalNAc targeting molecule.

Further, the ligase is a DNA T4 ligase.

In this Example, a sequence of the promoter is as shown in SEQ ID No. 3.

Further, a sequence of the target gene is as shown in SEQ ID No. 4.
SEQ ID No.3: taatacgactcactatagg;
SEQ ID No.4:

gtgagcaagggcgaggagaccacaatgggcgtaatcaagcccgacatgaagatcaagctgaagatg
gagggcaacgtgaatggccacgccttcgtgatcgagggcgagggcgagggcaagccctacgacggcacca
acaccatcaacctggaggtgaaggagggagcccccctgcccttctcctacgacattctgaccaccgcgttcagt
tacggcaacagggccttcaccaagtaccccgacgacatccccaactacttcaagcagtccttccccgagggct
actcttgggagcgcaccatgaccttcgaggacaagggcatcgtgaaggtgaagtccgacatctccatggagg
aggactccttcatctacgagatacacctcaagggcgagaacttcccccccaacggccccgtgatgcagaagg
agaccaccggctgggacgcctccaccgagaggatgtacgtgcgcgacggcgtgctgaagggcgacgtcaa
gatgaagctgctgctggagggcggcggccaccaccgcgttgacttcaagaccatctacagggccaagaagg
cggtgaagctgcccgactatcactttgtggaccaccgcatcgagatcctgaaccacgacaaggactacaacaa
ggtgaccgtttacgagatcgccgtggcccgcaactccaccgacggcatggacgagctgtacaagtaa.

The mRNA-GalNAc targeting molecule prepared in this Example can be used in preparation of an mRNA drug for specific drug delivery. The 3'-end of the mRNA-GalNAc targeting molecule is linked to an N-acetylgalactosamine, and the mRNA-GalNAc targeting molecule specifically binds to sialoglycoprotein receptors on a surface of the liver cell through the N-acetylgalactosamine, which induces endocytosis and therefore allows the mRNA to enter into the cell for expression.

### Example 2

Provided herein is an mRNA-GalNAc targeting molecule comprising an mRNA molecule, wherein a sequence of the mRNA molecule comprises a 5' cap and a target gene sequence; the mRNA molecule is linked to PolyA modified with an N-acetylgalactosamine at 3'-end through a splint DNA; and the mRNA-GalNAc targeting molecule comprises a positively charged protein molecule.

As shown in Figure 2, the mRNA-GalNAc targeting molecule is prepared by the following steps.
Step S1, designing and synthesizing a plasmid vector having a promoter sequence, a target gene sequence and a sequence complementary to a first splint DNA sequence;
Step S2, *in vitro* transcribing the plasmid vector of Step S1 as a template to obtain an mRNA molecule, wherein a sequence of the mRNA molecule comprise a 5'cap, the target gene sequence, and an RNA sequence corresponding to the sequence complementary to the first splint DNA sequence, that are sequentially connected; and
Step S3, the PolyA fragment modified with an N-acetylgalactosamine at 3'-end has a 5'-end which is an RNA sequence that can be complementarily paired with a second splint DNA sequence, wherein the first splint DNA sequence and the second splint DNA sequence form the splint DNA; in annealing reaction, the mRNA molecule with the sequence complementary to the first splint DNA sequence and the PolyA molecule with the sequence complementarily paired with the second splint DNA sequence complementarily bind to the splint DNA, respectively; a 3'-end hydroxyl group of the mRNA molecule is linked to a 5'-end phosphate group of the PolyA modified with an N-acetylgalactosamine under the action of a T4 DNA ligase; and after treatment by a DNase, the mRNA targeting molecule modified with an N-acetylgalactosamine is obtained.

In this Example, a sequence of the first splint DNA is as shown in SEQ ID No. 1, and a sequence of the second splint DNA is as shown in SEQ ID No. 2.

A sequence of the promoter is as shown in SEQ ID No. 3.

A sequence of the target gene is as shown in SEQ ID No. 4.

Further, the ligase is a DNA T4 ligase.

The mRNA-GalNAc targeting molecule obtained in this Example can be used in preparation of an mRNA drug for specific drug delivery. The 3'-end of the mRNA-GalNAc targeting molecule is linked to an N-acetylgalactosamine, and the mRNA-GalNAc targeting molecule specifically binds to sialoglycoprotein receptors on a surface of the liver cell through the N-acetylgalactosamine, which induces endocytosis and therefore allows the mRNA to enter into the cell for expression, as shown in Figures 3 and 4.

### Example 3

Provided herein is an *in vivo* delivery system, which comprises the mRNA-GalNAc targeting molecule of Example 2 and a positively charged protein molecule. After the mRNA-GalNAc targeting molecule was obtained, the *in vivo* delivery system was obtained by adjusting the charge property of the mRNA-GalNAc targeting molecule using adjuvants, protamine and/or human serum albumin (HSA). This step allowed the mRNA-GalNAc targeting molecule to contain a positively charged protein, which improved the stability and the transfection efficiency of the mRNA *in vivo.* Further, the positively charged protein was at least one of protamine and human serum albumin.

In this Example, by screening effective positively charged protein molecules and neutralizing the negative charge of the mRNA molecule itself, the stability of the mRNA-GalNAc *in vivo* was improved, the efficiency of escape from endosome was increased, thereby the tissue and organ targeting delivery *in vivo* was realized. The principle of action was as shown in Figure 4.

This Example also selected and used different ratios of protamine to human serum albumin, as well as protamine or human serum albumin alone for experiments. The molar Ratio A of protamine to HAS was 1:4, and the molar Ratio B of protamine to HAS was 1:12. The test results were as shown in Figures 5(a)-(c). It can be seen from comparison that, the delivery systems with the two mixed positively charged proteins of protamine and human serum albumin had better stability and transfection efficiency than the delivery systems with a single positively charged protein, and the delivery system with protamine and human serum albumin in Ratio A had the highest stability and transfection efficiency.

In addition, the delivery system with protamine and human serum albumin in Ratio A or Ratio B was compared experimentally to the delivery system without protamine and human serum albumin. As shown in Figure 6, the delivery system with Ratio A or Ratio B was more efficient than the delivery system without protamine and human serum albumin in term of expression of green fluorescent protein (GFP) in liver cells.

### Example 4

Provided herein is an *in vivo* delivery system based on Example 3, which comprises an mRNA-GalNAc targeting molecule and a positively charged protein, wherein the target gene sequence in the mRNA-GalNAc targeting molecule is as shown in SEQ ID No. 5, which is different from Example 3.

SEQ ID No.5:

atggaagatgccaaaaacattaagaagggcccagcgccattctacccactcgaagacgggaccgccg
gcgagcagctgcacaaagccatgaagcgctacgccctggtgcccggcaccatcgcctttaccgacgcacata
tcgaggtggacattacctacgccgagtacttcgagatgagcgttcggctggcagaagctatgaagcgctatgg
gctgaatacaaaccatcggatcgtggtgtgcagcgagaatagcttgcagttcttcatgcccgtgttgggtgccct
gttcatcggtgtggctgtggccccagctaacgacatctacaacgagcgcgagctgctgaacagcatgggcatc
agccagcccaccgtcgtattcgtgagcaagaaagggctgcaaaagatcctcaacgtgcaaaagaagctaccg
atcatacaaaagatcatcatcatggatagcaagaccgactaccagggcttccaaagcatgtacaccttcgtgact
tcccatttgccacccggcttcaacgagtacgacttcgtgcccgagagcttcgaccgggacaaaaccatcgccct
gatcatgaacagtagtggcagtaccggattgcccaagggcgtagccctaccgcaccgcaccgcttgtgtccga
ttcagtcatgcccgcgaccccatcttcggcaaccagatcatccccgacaccgctatcctcagcgtggtgccattt
caccacggcttcggcatgttcaccacgctgggctacttgatctgcggctttcgggtcgtgctcatgtaccgcttcg
aggaggagctattcttgcgcagcttgcaagactataagattcaatctgccctgctggtgcccacactatttagcttc
ttcgctaagagcactctcatcgacaagtacgacctaagcaacttgcacgagatcgccagcggcggggcgccg
ctcagcaaggaggtaggtgaggccgtggccaaacgcttccacctaccaggcatccgccagggctacggcct
gacagaaacaaccagcgccattctgatcacccccgaaggggacgacaagcctggcgcagtaggcaaggtg
gtgcccttcttcgaggctaaggtggtggacttggacaccggtaagacactgggtgtgaaccagcgcggcgag
ctgtgcgtccgtggccccatgatcatgagcggctacgttaacaaccccgaggctacaaacgctctcatcgacaa
ggacggctggctgcacagcggcgacatcgcctactgggacgaggacgagcacttcttcatcgtggaccggct
gaagagcctgatcaaatacaagggctaccaggtagccccagccgaactggagagcatcctgctgcaacaccc
caacatcttcgacgccggggtcgccggcctgcccgacgacgatgccggcgagctgcccgccgcagtcgtcg
tgctggaacacggtaaaaccatgaccgagaaggagatcgtggactatgtggccagccaggttacaaccgcca
agaagctgcgcggtggtgttgtgttcgtggacgaggtgcctaaaggactgaccggcaagttggacgcccgca
agatccgcgagattctcattaaggccaagaagggcggcaagatcgccgtgtaa.

After the mRNA-GalNAc targeting molecule was obtained, the *in vivo* delivery system was obtained by adjusting the charge property of the mRNA-GalNAc targeting molecule using adjuvants, protamine and HSA. The molar Ratio A of protamine to HAS was 1:4 and the molar Ratio B of protamine to HAS was 1:12.

The delivery system with protamine and human serum albumin in Ratio A or Ratio B was compared experimentally to the delivery system without protamine and human serum albumin. As shown in Figure 7, the delivery system with Ratio A or Ratio B was more efficient than the delivery system without protamine and human serum albumin in term of delivery of luciferase (Luc) to a liver tissue. The delivery system with protamine and human serum albumin in Ratio A had the best effect.

### Example 5

The EPO mRNAs with and without the Kozak sequence, as well as Luciferase mRNAs with and without GalNAc modification, were synthesized. 293T cells were cultured *in vitro* and transfected with the EPO mRNA, and the total protein in the 293T cells was extracted after 24 hours. The effect of the Kozak sequence on expression efficiency of the EPO mRNA was determined by Western blot assay. The Luciferase mRNA was injected into mice intramuscularly and after 24 hours, 200 ul of 15 mg/ml luciferin was injected intraperitoneally for three-dimensional color imaging. The effect of GalNAc modification on expression efficiency of the mRNA was compared based on luminescence intensities. The result was shown in Figure 8.

### Example 6

The Epo mRNA-GalNAc molecules were synthesized. The unmodified versions were consisted of uridine, cytosine, adenosine, guanosine and chemically modified nucleosides. The modified versions were those in which the original nucleotide molecule was replaced with one of 2-fluoro-2-deoxyadenosine, 2-fluoro-2-deoxyuridine, 2-fluoro-2-deoxycytidine, 2-fluoro-2-deoxyguanosine, 2-fluoro-2-deoxy-5-methylcytidine, 2-fluoro-2-deoxy-pseudouridine, 2-fluoro-2-deoxy-N1-methyl-pseudouridine, 2-fluoro-2-deoxy-N7-methyl-guanosine, 2-fluoro-2-deoxy-5-methoxyuridine, 2-fluoro-2-deoxy-N4-acetylcytidine, 2-fluoro-2-deoxy-N6-methyladenosine, 5-methylcytidine, pseudouridine, N1-methyl-pseudouridine, N7-methyl-guanosine, 5-methoxyuridine, N4-acetylcytidine and N6-methyladenosine.

All of the mRNA molecules were formulated into a 250 ug/ml solution in physiological saline. Each 8-week-old Balb/c mouse was injected with 200 ul of the solution through the tail vein. The peripheral blood serum of the mouse was collected after 24 hours for Elisa experiment to determine expression level of the Epo protein.

The experimental protocol was as follows.
Reagents: Anti-EPO antibody (ab226956), and Goat Anti-Rabbit IgG H&L (HRP) (ab6721).
Consumable materials: Greiner 96-well microtiter plates, pipette tips 25 ml, pipette tips 1 ml, and pipette tips 300ul.
Instruments and equipment: biotek Epch2 microplate reader, and Microplate SOTS Automatic Plate Washer.

### Experimental procedures:

Coating: The volume of the coating sample was 100 ul, and the mass of the coating standards was 2ng/0.2ng/0.02ng/0.002ng/0.0002ng/0.00002ng. A calculated amount of sample was taken and diluted in 100 ul of coating buffer, and then was added to the 96-well plate by multichannel pipettes. And then, the plate was covered with a microplate sealer and was allowed to stand at 4°C overnight.

Blocking: The coated 96-well plate was tilted to remove the coating buffer. Then the coated 96-well plate was placed upside down on an absorbent paper and shook until there was no residue in the wells.

Plate washing: The washing buffer was prepared and diluted with deionized water by 50 folds, and added to the liquid inlet bottle of the plate washer. The program was set, in which the volume of the washing buffer in each well was set to 300 µl and the washing was repeated four times.

Blocking: The washed plate was placed upside down and shook to remove the solution inside until dryness. Then the plate was added with the blocking buffer in a volume of 250 ul per well and covered with a microplate sealer, and allowed to stand at room temperature for 2 hours.

Plate washing: The blocked microtiter plate was washed according to step 3.

Incubation of Primary antibody: The primary antibody was diluted with a dilution buffer and added to the washed 96-well plate in a volume of 100 ul per well. The plate was covered with a microplate sealer and incubated at room temperature for 1.5 hours.

Plate washing: The plate was washed according to step 3, and the washing was repeated 6 times.

Addition of secondary antibody: HRP-labeled goat anti-rabbit IgG was diluted with the dilution buffer at dilution factor of 10000 and the diluted antibody was added to the microtiter plate in a volume of 100 ul per well. The plate was covered with a microplate sealer and incubated for 1 hour at room temperature in the dark.

Plate washing: The plate was washed according to step 8. In this step, the plate must be washed thoroughly and placed upside down to remove the solution until dryness.

Color development: 100 ul of TMB buffer was added. The color development was performed in the dark for 20-30 minutes. At this time, the positive sample exhibited blue color.

Termination: 100 ul of a stop buffer was added. The reading was performed on the microtiter plate within 10 minutes, and the absorption wavelength was set to 450 nm.

The experimental results were as shown in Figure 9. The chemical modification of bases can significantly increase the expression level of mRNA-GalNAc molecules.

## Claims

1. An mRNA-N-acetylgalactosamine (GalNAc) targeting molecule comprising an mRNA molecule and a GalNAc, wherein the mRNA molecule comprises a target gene sequence, and wherein the GalNAc is directly conjugated to a polyA sequence of the mRNA molecule.

2. The mRNA-GalNAc targeting molecule of claim 1, wherein the mRNA-GalNAc targeting molecule comprises a 5' cap, and wherein the 5' cap comprises one or more of Cap0, Cap1, Cap2, ARCA, inosine, N1-methyl-guanosine, 2'fluoro-guanosine, 7-deaza-guanosine, 8-oxo-guanosine, 2-amino-guanosine, LNA-guanosine, 2-azido-guanosine, 7-methyl-guanosine-5'-triphosphate-5'-adenosine, guanosine-5'-triphosphate-5'-adenosine, 7-methyl-guanosine-5'-triphosphate-5'-guanosine, guanosine-5'-triphosphate-5'-guanosine, and 7-methyl-guanosine-5'-triphosphate-5'-2-methoxyadenine-guanosine.

3. The mRNA-GalNAc targeting molecule of claim 1 or 2, wherein the mRNA molecule comprises one or more of a chemically modified nucleoside, wherein the chemically modified nucleoside comprises 2-fluoro-2-deoxyadenosine, 2 -fluoro-2-deoxyuridine, 2-fluoro-2-deoxycytidine, 2-fluoro-2-deoxyguanosine, 2-fluoro-2-deoxy-5-methylcytidine, 2-fluoro-2-deoxy-pseudouridine, 2-fluoro-2-deoxy-N1-methyl-pseudouridine, 2-fluoro-2-deoxy-N7-methyl-guanosine, 2-fluoro-2-deoxy-5-methoxyuridine, 2-fluoro-2-deoxy-N4-acetylcytidine, 2-fluoro-2-deoxy-N6-methyladenosine, 5-methylcytidine, pseudouridine, N1-methyl-pseudouridine, N7-methyl-guanosine, 5-methoxyuridine, N4-acetylcytidine or N6-methyladenosine.

4. The mRNA-GalNAc targeting molecule of any one of the preceding claims, wherein the mRNA molecule comprises a 5' UTR and/or a 3' UTR, and optionally wherein the 5' UTR comprises a Kozak sequence.

5. An *in vivo* delivery system comprising the mRNA-GalNAc targeting molecule of any one of claims 1 to 4 and a positively charged protein molecule.

6. The *in vivo* delivery system of claim 5, wherein the positively charged protein molecule comprises protamine and/or human serum albumin, and optionally wherein a molar ratio of the protamine to the human serum albumin is 1: (2.75-5.5) or 1: (6-20).

7. A method of preparing an mRNA-N-acetylgalactosamine (GalNAc) targeting molecule comprising an mRNA molecule and a GalNAc, wherein the method comprises:
(a). *in vitro* transcribing from a plasmid vector the mRNA molecule, wherein the plasmid vector comprises a promoter sequence and a target gene sequence, and wherein the mRNA is transcribed from the target gene sequence; and
(b). conjugating the mRNA molecule and a polyA sequence modified with a GalNAc at 3'-end with a ligase, optionally wherein the ligase is a T4 ligase,
thereby obtaining the mRNA-GalNAc targeting molecule.

8. The method of claim 7, wherein the plasmid vector comprises a 5' UTR and/or a 3' UTR, optionally wherein the 5' UTR comprises a Kozak sequence, wherein the promoter is T3, T7, or SP6, and optionally wherein the promoter comprises a sequence set forth in SEQ ID NO: 3.

9. The method of any one of claims 7 or 8, wherein in (b) a 3'-end hydroxyl group of the mRNA molecule is linked to a 5'-end phosphate group of the polyA sequence.

10. A method of preparing an mRNA-N-acetylgalactosamine (GalNAc) targeting molecule comprising an mRNA molecule and a GalNAc, wherein the method comprises:
(a). *in vitro* transcribing from a plasmid vector the mRNA molecule, wherein the plasmid vector comprises a promoter sequence, a target gene sequence, and a sequence that is complementary to a first splint DNA sequence, and wherein the mRNA is transcribed from the target gene sequence;
(b). providing a moiety, wherein the moiety comprises, from its 5'end to 3' end, an RNA sequence complementary to a second splint DNA sequence, a polyA sequence, and the GalNAc; and
(c). conjugating the mRNA molecule and the moiety with a ligase and a splint DNA, and the splint DNA comprises the first splint DNA sequence and the second splint DNA sequence, optionally wherein the ligase is a T4 ligase
thereby obtaining the mRNA-GalNAc targeting molecule.

11. The method of claim 10, wherein the first splint DNA sequence comprises a sequence set forth in SEQ ID NO: 1, and wherein the second splint DNA sequence comprises a sequence set forth in SEQ ID NO: 2.

12. The method of any one of claims 10 to 11, wherein the plasmid vector comprises a 5' UTR and/or a 3' UTR, optionally wherein the 5' UTR comprises a Kozak sequence, wherein the promoter is T3, T7, or SP6, and optionally wherein the promoter comprises a sequence set forth in SEQ ID NO: 3.

13. The method of any one of claims 10 to 12, wherein (c) is carried out in an annealing reaction.

14. The method of any one of claims 10 to 13, wherein in (c) a 3'-end hydroxyl group of the mRNA molecule is linked to a 5'-end phosphate group of the polyA sequence.

15. The mRNA-GalNAc targeting molecule of any one of claims 1 to 4, or the *in vivo* delivery system of any one of claims 5 to 6 for use in a method of expressing a target gene sequence in a subject, wherein the method comprises delivering said targeting molecule or system to the subject.

16. The molecule or system for use of claim 15, wherein the delivering is carried out intravenously or intramuscularly.

## Patentansprüche

1. mRNA-N-Acetylgalactosamin(GaINAc)-Targeting-Molekül, das ein mRNA-Molekül und ein GalNAc umfasst, wobei das mRNA-Molekül eine Zielgensequenz umfasst und wobei das GalNAc direkt an eine PolyA-Sequenz des mRNA-Moleküls konjugiert ist.

2. mRNA-GalNAc-Targeting-Molekül nach Anspruch 1, wobei das mRNA-GalNAc-Targeting-Molekül eine 5'-Kappe umfasst und wobei die 5'-Kappe eines oder mehrere aus Cap0, Cap1, Cap2, ARCA, Inosin, N1-Methylguanosin, 2'-Fluorguanosin, 7-Deazaguanosin, 8-Oxoguanosin, 2-Aminoguanosin, LNA-Guanosin, 2-Azidoguanosin, 7-Methylguanosin-5'-triphosphat-5'-adenosin, Guanosin-5'-triphosphat-5'-adenosin, 7-Methylguanosin-5'-triphosphat-5'-guanosin, Guanosin-5'-triphosphat-5'-guanosin und 7-Methylguanosin-5'-triphosphat-5'-2-methoxyadeninguanosin umfasst.

3. mRNA-GalNAc-Targeting-Molekül nach Anspruch 1 oder 2, wobei das mRNA-Molekül ein oder mehrere chemisch modifizierte Nucleoside umfasst, wobei das chemisch modifizierte Nucleosid 2-Fluor-2-desoxyadenosin, 2-Fluor-2-desoxyuridin, 2-Fluor-2-desoxycytidin, 2-Fluor-2-desoxyguanosin, 2-Fluor-2-desoxy-5-methylcytidin, 2-Fluor-2-desoxypseudouridin, 2-Fluor-2-desoxy-N1-methylpseudouridin, 2-Fluor-2-desoxy-N7-methylguanosin, 2-Fluor-2-desoxy-5-methoxyuridin, 2-Fluor-2-desoxy-N4-acetylcytidin, 2-Fluor-2-desoxy-N6-methyladenosin, 5-Methylcytidin, Pseudouridin, N1-Methylpseudouridin, N7-Methylguanosin, 5-Methoxyuridin, N4-Acetylcytidin oder N6-Methyladenosin umfasst.

4. mRNA-GalNAc-Targeting-Molekül nach einem der vorangegangenen Ansprüche, wobei das mRNA-Molekül eine 5'-UTR und/oder eine 3'-UTR umfasst und wobei die 5'-UTR gegebenenfalls eine Kozak-Sequenz umfasst.

5. In-vivo-Abgabesystem, das ein mRNA-GalNAc-Targeting-Molekül nach einem der Ansprüche 1 bis 4 und ein positiv geladenes Proteinmolekül umfasst.

6. In-vivo-Abgabesystem nach Anspruch 5, wobei das positiv geladene Proteinmolekül Protamin und/oder Humanserumalbumin umfasst und wobei das molare Verhältnis zwischen dem Protamin und dem Humanserumalbumin gegebenenfalls 1:(2,75-5,5) oder 1:(6-20) beträgt.

7. Verfahren zur Herstellung eines mRNA-N-Acetylgalactosamin(GaINAc)-Targeting-Moleküls, das ein mRNA-Molekül und ein GalNAc umfasst, wobei das Verfahren Folgendes umfasst:
(a). In-vitro-Transkribieren des mRNA-Moleküls aus einem Plasmidvektor, wobei der Plasmidvektor eine Promotorsequenz und eine Zielgensequenz umfasst und wobei die mRNA aus der Zielgensequenz transkribiert wird; und
(b). Konjugieren des mRNA-Moleküls und einer PolyA-Sequenz, die mit einem GalNAc modifiziert ist, an das 3'-Ende mit einer Ligase, wobei die Ligase gegebenenfalls eine T4-Ligase ist,
wodurch das mRNA-GalNAc-Targeting-Molekül erhalten wird.

8. Verfahren nach Anspruch 7, wobei der Plasmidvektor eine 5'-UTR und/oder eine 3'-UTR umfasst, wobei die 5'-UTR gegebenenfalls eine Kozak-Sequenz umfasst, wobei der Promotor T3, T7 oder SP6 ist und wobei der Promotor gegebenenfalls eine in SEQ ID NO: 3 dargelegte Sequenz umfasst.

9. Verfahren nach einem der Ansprüche 7 oder 8, wobei in (b) eine Hydroxylgruppe am 3'-Ende des mRNA-Moleküls an eine Phosphatgruppe am 5'-Ende der PolyA-Sequenz gebunden ist.

10. Verfahren zur Herstellung eines mRNA-N-Acetylgalactosamin(GaINAc)-Targeting-Moleküls, das ein mRNA-Molekül und ein GalNAc umfasst, wobei das Verfahren Folgendes umfasst:
(a). In-vitro-Transkribieren des mRNA-Moleküls aus einem Plasmidvektor, wobei der Plasmidvektor eine Promotorsequenz, eine Zielgensequenz und eine Sequenz umfasst, die komplementär zu einer ersten Splint-DNA-Sequenz ist, und wobei die mRNA aus der Zielgensequenz transkribiert wird; und
(b). Bereitstellen einer Gruppierung, wobei die Gruppierung vom 5'-Ende zum 3'-Ende eine RNA-Sequenz, die komplementär zu einer zweiten Splint-DNA-Sequenz ist, eine PolyA-Sequenz und das GalnAc umfasst; und
(c). Konjugieren des mRNA-Moleküls und der Gruppierung mit einer Ligase und einer Splint-DNA, wobei die Splint-DNA die erste Splint-DNA-Sequenz und die zweite Splint-DNA-Sequenz umfasst, wobei die Ligase gegebenenfalls eine T4-Ligase ist,
wodurch das mRNA-GalNAc-Targeting-Molekül erhalten wird.

11. Verfahren nach Anspruch 10, wobei die erste Splint-DNA-Sequenz eine in SEQ ID NO: 1 dargelegte Sequenz umfasst und wobei die zweite Splint-DNA-Sequenz eine in SEQ ID NO: 2 dargelegte Splint-Sequenz umfasst.

12. Verfahren nach einem der Ansprüche 10 bis 11, wobei der Plasmidvektor eine 5'-UTR und/oder eine 3'-UTR umfasst, wobei die 5'-UTR gegebenenfalls eine Kozak-Sequenz umfasst, wobei der Promotor T3, T7 oder SP6 ist und wobei der Promotor gegebenenfalls eine in SEQ ID NO: 3 dargelegte Sequenz umfasst.

13. Verfahren nach einem der Ansprüche 10 bis 12, wobei (c) in einer Annealing-Reaktion ausgeführt wird.

14. Verfahren nach einem der Ansprüche 10 bis 13, wobei in (c) eine Hydroxylgruppe am 3'-Ende des mRNA-Moleküls an eine Phosphatgruppe am 5'-Ende der PolyA-Sequenz gebunden ist.

15. mRNA-GalNAc-Targeting-Molekül nach einem der Ansprüche 1 bis 4 oder In-vivo-Abgabesystem nach einem der Ansprüche 5 bis 6 zur Verwendung in einem Verfahren zur Expression einer Zielgensequenz in einem Individuum, wobei das Verfahren die Abgabe des Targeting-Moleküls oder Systems an das Individuum umfasst.

16. Molekül oder System zur Verwendung nach Anspruch 15, wobei die Abgabe intravenös oder intramuskulär erfolgt.

## Revendications

1. Molécule de ciblage d'ARNm-N-acétylgalactosamine (GalNAc) comprenant une molécule d'ARNm et une GalNAc, dans laquelle la molécule d'ARNm comprend une séquence de gène cible, et dans laquelle la GalNAc est directement conjuguée à une séquence polyA de la molécule d'ARNm.

2. Molécule de ciblage d'ARNm-GalNAc selon la revendication 1, dans laquelle la molécule de ciblage d'ARNm-GalNAc comprend une coiffe en 5', et dans laquelle la coiffe en 5' comprend un ou plusieurs parmi Cap0, Cap1, Cap2, ARCA, inosine, N1-méthyl-guanosine, 2'fluoro-guanosine, 7-déaza-guanosine, 8-oxo-guanosine, 2-amino-guanosine, LNA-guanosine, 2-azido-guanosine, 7-méthyl-guanosine-5'-triphosphate-5'-adénosine, guanosine-5'-triphosphate-5'-adénosine, 7-méthyl-guanosine-5'-triphosphate-5'-guanosine, guanosine-5'-triphosphate-5'-guanosine, et 7-méthyl-guanosine-5'-triphosphate-5'-2-méthoxyadénine-guanosine.

3. Molécule de ciblage d'ARNm-GalNAc selon la revendication 1 ou 2, dans laquelle la molécule d'ARNm comprend un ou plusieurs d'un nucléoside chimiquement modifié, dans laquelle le nucléoside chimiquement modifié comprend de la 2-fluoro-2-désoxyadénosine, 2-fluoro-2-désoxyuridine, 2-fluoro-2-désoxycytidine, 2-fluoro-2-désoxyguanosine, 2-fluoro-2-désoxy-5-méthylcytidine, 2-fluoro-2-désoxy-pseudouridine, 2-fluoro-2-désoxy-N1-méthyl-pseudouridine, 2-fluoro-2-désoxy-N7-méthyl-guanosine, 2-fluoro-2-désoxy-5-méthoxyuridine, 2-fluoro-2-désoxy-N4-acétylcytidine, 2-fluoro-2-désoxy-N6-méthyladénosine, 5-méthylcytidine, pseudouridine, N1-méthyl-pseudouridine, N7-méthyl-guanosine, 5-méthoxyuridine, N4-acétylcytidine ou N6-méthyladénosine.

4. Molécule de ciblage d'ARNm-GalNAc selon l'une quelconque des revendications précédentes, dans laquelle la molécule d'ARNm comprend une région 5' UTR et/ou 3' UTR, et facultativement dans laquelle 5' UTR comprend une séquence Kozak.

5. Système d'administration *in vivo* comprenant la molécule de ciblage d'ARNm-GalNAc selon l'une quelconque des revendications 1 à 4 et une molécule protéique chargée positivement.

6. Système d'administration *in vivo* selon la revendication 5, dans lequel la molécule protéique chargée positivement comprend de la protamine et/ou de la sérumalbumine humaine, et facultativement dans lequel un rapport molaire entre la protamine et la sérumalbumine humaine est de 1:(2,75 à 5,5) ou de 1:(6 à 20).

7. Procédé de préparation d'une molécule de ciblage d'ARNm-N-acétylgalactosamine (GalNAc) comprenant une molécule d'ARNm et une GalNAc, dans lequel le procédé comprend les étapes consistant à :
(a). transcrire *in vitro* à partir d'un vecteur plasmidique la molécule d'ARNm, dans lequel le vecteur plasmidique comprend une séquence promotrice et une séquence de gène cible, et dans lequel l'ARNm est transcrit à partir de la séquence de gène cible ; et
(b). conjuguer la molécule d'ARNm et une séquence polyA modifiée avec une GalNAc à l'extrémité 3' avec une ligase, facultativement dans lequel la ligase est une ligase T4,
obtenant ainsi la molécule de ciblage d'ARNm-GalNAc.

8. Procédé selon la revendication 7, dans lequel le vecteur plasmidique comprend une région 5' UTR et/ou 3' UTR, facultativement dans lequel 5' UTR comprend une séquence Kozak, dans lequel le promoteur est T3, T7, ou SP6, et facultativement dans lequel le promoteur comprend une séquence présentée dans SEQ ID NO : 3.

9. Procédé selon l'une quelconque des revendications 7 ou 8, dans lequel lors de (b), un groupe hydroxyle d'extrémité 3' de la molécule d'ARNm est lié à un groupe phosphate d'extrémité 5' de la séquence polyA.

10. Procédé de préparation d'une molécule de ciblage ARNm-N-acétylgalactosamine (GalNAc) comprenant une molécule d'ARNm et une GalNAc, dans lequel le procédé comprend les étapes consistant à :
(a). transcrire *in vitro* à partir d'un vecteur plasmidique la molécule d'ARNm, dans lequel le vecteur plasmidique comprend une séquence promotrice, une séquence de gène cible et une séquence qui est complémentaire d'une première séquence d'ADN attelle, et dans lequel l'ARNm est transcrit à partir de la séquence de gène cible ;
(b). fournir un fragment, dans lequel le fragment comprend, de son extrémité 5' à son extrémité 3', une séquence d'ARN complémentaire d'une seconde séquence d'ADN attelle, une séquence polyA et la GalNAc ; et
(c). conjuguer la molécule d'ARNm et le fragment avec une ligase et un ADN attelle, et l'ADN attelle comprend la première séquence d'ADN attelle et la seconde séquence d'ADN attelle, facultativement, la ligase étant une ligase T4
obtenant ainsi la molécule de ciblage d'ARNm-GalNAc.

11. Procédé selon la revendication 10, dans lequel la première séquence d'ADN attelle comprend une séquence présentée dans SEQ ID NO : 1, et dans lequel la seconde séquence d'ADN attelle comprend une séquence présentée dans SEQ ID NO : 2.

12. Procédé selon l'une quelconque des revendications 10 à 11, dans lequel le vecteur plasmidique comprend une région 5' UTR et/ou 3' UTR, facultativement dans lequel 5' UTR comprend une séquence Kozak, dans lequel le promoteur est T3, T7, ou SP6, et dans lequel facultativement le promoteur comprend une séquence présentée dans SEQ ID NO : 3.

13. Procédé selon l'une quelconque des revendications 10 à 12, dans lequel (c) est effectué dans une réaction de recuit.

14. Procédé selon l'une quelconque des revendications 10 à 13, dans lequel lors de (c), un groupe hydroxyle d'extrémité 3' de la molécule d'ARNm est lié à un groupe phosphate d'extrémité 5' de la séquence polyA.

15. Molécule de ciblage d'ARNm-GalNAc selon l'une quelconque des revendications 1 à 4, ou système d'administration *in vivo* selon l'une quelconque des revendications 5 à 6 pour une utilisation dans un procédé d'expression d'une séquence de gène cible chez un sujet, dans lequel le procédé comprend l'administration au sujet de ladite molécule de ciblage ou dudit système.

16. Molécule ou système pour une utilisation selon la revendication 15, dans lequel l'administration est effectuée par voie intraveineuse ou intramusculaire.
